# EUROPEAN PATENT SPECIFICATION

(11) **EP 1 542 735 B1**
(45) Date of publication and mention of the grant of the patent: **21.03.2012**
(21) Application number: 03748300.5
(22) Date of filing: 23.09.2003
(51) Int. Cl.: A61L 2/20, A61L 2/26

(54) **A PRE-STERILISATION CHAMBER FOR A PROCESSING ENCLOSURE**
EINE VOR-STERILISIERUNGSKAMMER FÜR EINEN GESCHLOSSENEN VERARBEITUNGSRAUM
CHAMBRE DE PRE-STERILISATION POUR ENCEINTE DE TRAITEMENT

(30) Priority: 24.09.2002 GB 0222154
(43) Date of publication of application: 22.06.2005
(73) Proprietor: Bioquell UK Limited, West Portway Andover Hampshire SP10 3TS (GB)
(72) Inventor: Bissell, Donald Kerr, Bookham, Surrey KT23 3PZ (GB); Drinkwater, James Lindsay, Aylesbury, Buckinghamshire HP18 0BA (GB)
(74) Representative: Bayliss, Geoffrey Cyril
(86) International application number: PCT/GB2003/004087
(87) International publication number: WO 2004/028573

(56) References cited:
- JP-A- 2001 340 432
- US-A- 4 435 194
- US-A- 5 251 423
- US-A- 5 792 435
- US-A- 5 988 235

## Description

This invention relates to pre-sterilisation ante-chamber for processing enclosures such as isolator enclosures, rooms, cabinets or the like in which processing operations are conducted under sterile conditions.

US-A-5,251,423 discloses a filling and closing machine for the packaging of comestibles in plastic cups. The machine has a sterilising chamber in which the cups are subjected to contact with H₂O₂ vapour followed by hot sterile air for the final sterilisation and a pre-sterilisation chamber in which the cups are contacted with H₂O₂ containing air from the sterilisation chamber.

US-A-5,988,235 discloses an apparatus for sterilising, filling and sealing containers comprising a sterilisation chamber, a cooling chamber and a filling and sealing chamber through which the containers are conveyed by means of an endless conveyor device. The chambers are pre-sterilised by gaseous sterilising agent which flows through them through the inlet or outlet for the containers. A reversible blower is provided for delivering the sterilisation agent through the chambers, the direction being periodically reversed a multiplicity of times to ensure that regions of the chambers less affected by the sterilising agent are well disinfected.

US-A-4,435,194 discloses a circuit for ventilating and filtering a medium contained in a confinement enclosure. A standby circuit is associated with the circuit which ensures ventilation and filtration under normal operating conditions and which comprises a supply pipe and a discharge pipe equipped with a suction fan. The standby circuit comprises the same pipes used in the opposite direction under normal conditions under the action of a suction fan located in the supply pipe whereby the fan can be automatically controlled by a pressure responsive device. The sterilisation is performed chemically using a steriliser which is connected to the supply duct between a valve and the supply filters disposed downstream of the valve. Once sterilisation has been performed, the ventilating circuit is started up to sweep away the sterilising agent.

US-A-5,792,435 discloses a flow-through vapour phase decontamination apparatus which includes at least one isolator unit defining an isolated chamber that receives a load for decontamination. The apparatus includes a vapour phase decontamination generation system integrally connected to the at least isolator unit. The vapour phase decontaminant generator system includes one or more vaporisers that inject a combination of a carrier gas and a vaporised decontaminant such as H₂O₂ into the isolation chamber. A blower recirculates the carrier gas through one or more vaporisers for replenishment of the decontaminant vapour.

Typically when small numbers of aseptic drug preparations are required they are dispensed either in a hospital pharmacy or a pharmacy facility that serves a hospital. Normally the components and material required for the dispensing are placed in an isolator for aseptic processing. The surfaces inside the isolator are bio-decontaminated, generally by using a gaseous process; the drugs are then dispensed and removed from the isolator. The problem with this technique is that because the sterilising cycle is long, it is necessary to place sufficient components and material inside the isolator for one whole day's work. The workload must therefore be planned the previous day making it difficult to respond to emergencies and changes in requirements, making the process very inflexible. Large banks of pre-sterilised material are often therefore used to improve the flexibility of response, but this approach is space consuming and expensive, with long recovery times in the event of loss of sterility of the bank isolator.

The main reason that the decontamination process is long is the absorption of the sterilizing gas into the surface of the components and material forming the load and also the surfaces of the chamber including the HEPA filters used to provide a stream of sterile air to the chamber. If the size of the load can be reduced and HEPA filters not exposed to the gas during routine process transfer much shorter cycles times would result, thus giving the required flexibility to bio-decontaminate components and material on demand. Removing the HEPA filters from the space that is bio-decontaminated with the components and material creates a further problem, in that all surfaces that come into contact with the air entering or leaving the chamber must be sterile, or these surfaces will be a source of bio-contamination that may enter the chamber and hence contaminate the product.

JP 2001-340432 describes ozone sterilisation equipment which has a chamber connected to one line for introducing the ozone gas into the chamber, a second line which enables air to be introduced into the chamber and a single exhaust line for exhausting the gas and/or air from the chamber. Sterilisation filters are provided in the aeration line to filter the air. Valves are provided to enable ozone to be passed by means of a bypass line to an upstream side of the sterilisation filter in the aeration line.

The object of the invention is to provide rapid surface gaseous sterilisations of components and material within a chamber so that the surfaces of the said components and materials may be rendered sterile. The components and materials may then be transferred from the chamber into a sterile processing area without the risk of causing contamination within the processing area.

The invention provides an ante-chamber for pre-sterilising components/materials to be supplied to a processing enclosure, the ante-chamber having a closable entry for receipt of components/materials, a closable exit for supply of materials/components to the processing enclosure, and first valves for controlling the supply and return of sterilant vapour to and from the ante-chamber for sterilising the ante-chamber and its contents; characterised in that the ante-chamber has second valves for controlling the supply and return of purge gas to and from the ante-chamber via supply and return conduits for purging the ante-chamber of sterilant at the end of the sterilising operation, the supply and return conduits having filters to filter out particles from the purge gas being delivered to and recovered from the ante-chamber respectively, said second valves being disposed between the filters and ante-chamber; the arrangement being such that the first and second valve means are operable to periodically provide a flow of sterilant vapour to sterilise the purge gas supply and return conduits and filters.

A greater degree of flexibility is achieved by using a relatively small chamber on the side of the dispensing isolator, and devising a rapid surface sterilization process for the product and components inside the chamber. By reducing the sterilization time to less than 20 minutes it becomes possible to generate a flow of material through the small chamber into the working isolator and thus give a greater degree of flexibility to the operations. To achieve such a short cycle time it is essential to arrange that surface decontamination is achieved in about 6 minutes and that aeration, the removal of the sterilant gas is achieved in 14 minutes.

Surface sterilization will only be achieved in such a short period if the gas injection rate is high and the gas distribution within the chamber is carefully managed to achieve even gas distribution at even gas temperatures.

To achieve rapid aeration, high purge air rates are required but of equal importance is to ensure that there are no absorbent surfaces, such as HEPA filters, in contact with the gas, during the load sterilisation.

Preferably a valve controlled supply of sterilant is provided for the purge gas supply conduit for supplying sterilant vapour through the conduit and to the return conduit via the ante-chamber to sterilise the purge gas supply and return conduit.

In the latter case the valve for controlling the supply of sterilant to the purge gas supply conduit may be located upstream of the filter in the conduit.

In either of the latter arrangements the return conduit for purge gas from the chamber may have a catalyst downstream of the filter for converting the sterilant into products which may be discharged to atmosphere.

More specifically a further filter may be located in the return conduit downstream of the catalyst to remove any particle in the purge gas received from the catalyst.

In any of the above arrangements the sterilant gas supply conduit chamber may have a fan for delivering air to the ante-chamber via the filter and valve to purge sterilant gas from the chamber.

Also in any of the above arrangements, the return conduit for purge gas may have a fan for extracting purge gas from the chamber disposed downstream of the valve control and filter.

Furthermore the supply and return conduits for purge gas both contain a pair of filters and a catalyst for converting sterilant to harmless products disposed between the filters and the valves are arranged to open both return and supply conduits to atmosphere for delivery of sterilant gas from the ante-chamber to sterilise the supply and return conduits.

The following is a description of some specific embodiments of the invention, reference being made to the accompanying drawing in which:
Figure 1 is a diagrammatic illustration of an ante-chamber for pre-sterilising components/material before entry to a sterile processing enclosure; and
Figure 2 is a diagrammatic illustration of the ante-chamber of Figure 1 embodied in a closed loop system.

The components and material, known as the load, to be bio-decontaminated are placed inside a chamber 10 through a first chamber door 11. At the other end of the chamber 10 is a second door 12 connected to a dispensing isolator (not shown) or processing enclosure. It is preferred that the first and second doors are provided with interlocks such that only one door may be opened at a time and also so that a door may only be opened when the atmosphere inside the chamber 10 is safe. Indication lamps are provided adjacent each door to indicate the state of opening/closure of the doors.

Once the load is placed inside the chamber and the first and second doors are closed and sealed, sterilizing gas is introduced into the chamber via a port 13 which connected through a valve 14 to the chamber. At this time the valve 14 must be opened to allow the gas to enter the chamber. The sterilizing gas is removed from the chamber through a port 15 controlled by a valve 16. The most commonly used sterilizing gas is hydrogen peroxide, and generally the commercially available hydrogen peroxide gas generators operate as a closed loop system with the gas returning to the generator.

During the circulation of the sterilizing gas further valves 17 and 18 which are connected to the chamber remain closed. Once the gaseous sterilization phase has been completed and it is required to remove the gas from the chamber the valves 17 and 18 are opened and fans 19 and 20 are switched on. At this point a 3-way valve 21 is set to deliver air from fan 20 to the valve 18.

The fan 20 takes air from the surrounding environment passing it through the 3-way valve 21 and a HEPA filter 22 and valve 18 into the chamber. This fresh air will reduce the gas concentration in the chamber by dilution. An equal quantity of air must be removed from the chamber through the valve 17, HEPA filter 23, a catalytic filter 24 and a further HEPA filter 25, by fan 19. It is important that the air fed into the chamber by fan 20 is filtered through the HEPA filter 22 to ensure that the chamber and the load inside the chamber remains sterile after gassing. Also on the exhaust side the air removed from the chamber must pass firstly through a HEPA filter 23 to stop any particles escaping back into the chamber and rendering it non-sterile. The catalytic filter 24 is used to render the exhaust gas safe before it is passed through the further HEPA filter 25 to remove any dust particles and then back into the surrounding environment.

A further connection 26 to the chamber is required for a pressure transducer 27 to monitor the pressure inside the chamber. A small HEPA filter (not shown) in the connection 26 avoids any contamination of the chamber from the connection. The pressure as measured by the transducer 27 is used to control fans 19 and 20 to achieve the required pressure in the chamber. Fans 19 and 20 are adjusted to achieve an air flow through the chamber at sufficiently high flow rate to remove the sterilizing gas in about 15 minutes. Experiment has shown that this will require an air change rate of about 2000 per hour.

Because of the need to ensure that the hydrogen peroxide gas does not come into contact with the HEPA filters 22 and 23 there is a space in the conduit between the filter 23 and valve 17, and also a further space between the filter 22 and valve 18 which is not sterilized. This space forms part of the air path during the aeration cycle. Any contamination in these spaces may therefore be transferred to the chamber and hence may contaminate the load within the chamber.

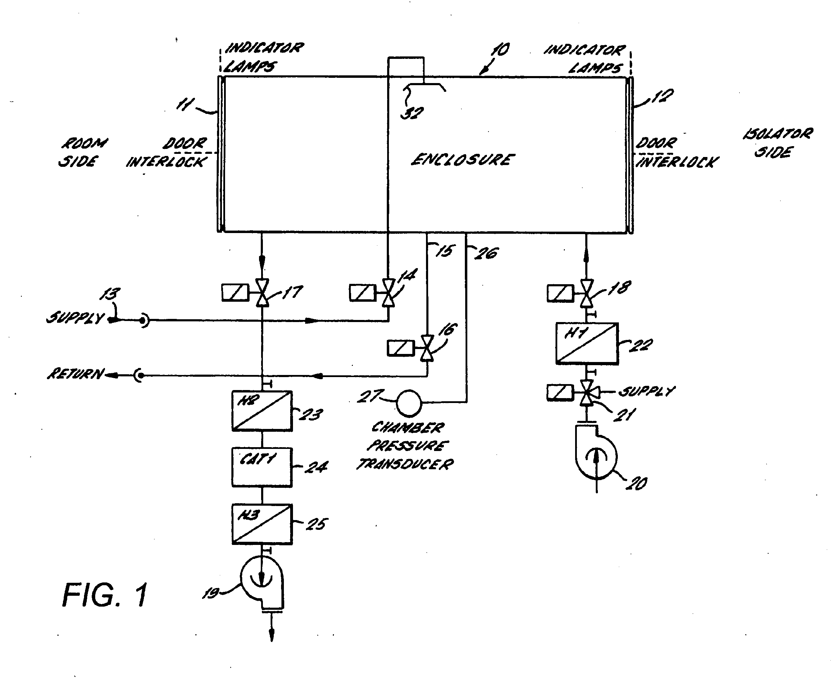

Two possible techniques are available to ensure that these spaces are bio-decontaminated and hence do not pose a risk to the load. The first will now be described by reference to Figure 1. The hydrogen peroxide gas supply is connected to the 3-way valve 21 such that the gas flows into the valve and thence to the chamber via the HEPA filter 22 and the valve 18, which must be open. The valves 14 and 16 are closed and the valve 17 opened to allow the gas to pass out through the HEPA filter 23, the carbon filter/catalyst 24 which renders the gas safe, through the HEPA filter 25 and finally exhausting through the fan 19. The passage of gas from the 3 way valve 21 through the chamber 10 and out through the fan 19 is allowed to continue for sufficient length of time to ensure decontamination of all of the components in this flow path.

At the end of the period the system is put back into aeration, as before, to remove the hydrogen peroxide vapour. Because this air path is protected by HEPA filtration it will require bio-decontamination at infrequent intervals, probably once every two weeks, depending on the usage of the chamber.

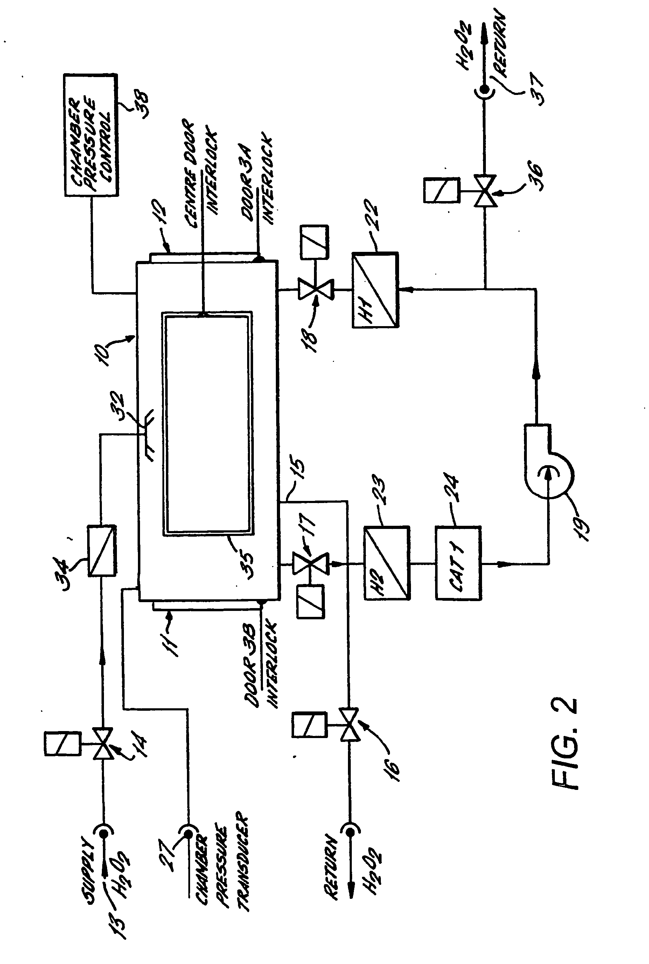

Reference is now made to Figure 2 which shows a closed loop system. The numbering system of Figure 1 is utilised in Figure 2, like parts being allotted the same reference numerals. The closed loop system avoids the need to exhaust air during the aeration phase. This has the advantage that should there be a failure in the catalytic destruction of the active gas then no toxic gas would be released into the room or environment. It also simplifies leak testing of the system as the number of potential leak paths is reduced.

The chamber of Figure 2 has up to three doors. One 11, 12 on each end as before to allow connection to two isolators and a third 35 in the centre through which the components to be sterilised are loaded. Each of these doors is fitted with a sensor to indicate when they are open or closed and a mechanism to ensure that only one is open at any time.

The gassing (sterilisation) process is the same as in Figure 1. Biodecontamination of the aeration pathway that is not sterilised during normal gassing is achieved by closing valves 14 and 16 and opening valves 17, 18 and 36. The gas supply is then connected to valve 18 and the return 37 to valve 17. This causes the sterilising gas to pass from the chamber through valve 17 and HEPA filter 23 thus exposing those surfaces not exposed to gas during the normal cycles.

Following the gassing cycle valves 17 and 18 are opened and the fan 19 is started. This generates a large air flow through the filter 23 and the catalytic destructor 24 that renders the active gas safe. After passing through the fan the air passes through a second HEPA filter 22 to remove any particulate contamination that may have arisen from the catalytic destructor or the fan. Because of the very high air flow (approximately 2000 to 3000 air changes per hour) through the catalytic destructor 24 the gas concentration in the chamber 10 is rapidly reduced to a safe level.

## Claims

1. An ante-chamber (10) for pre-sterilising components/materials to be supplied to a processing enclosure, the ante-chamber (10) having a closable entry (11) for receipt of components/materials, a closable exit (12) for supply of materials/components to the processing enclosure, and first valves (14,16) for controlling the supply and return of sterilant vapour to and from the ante-chamber (10) for sterilising the ante-chamber (10) and its contents; **characterised in that** the ante-chamber (10) has second valves (17,18) for controlling the supply and return of purge gas to and from the ante-chamber (10) via supply and return conduits for purging the ante-chamber (10) of sterilant at the end of the sterilising operation, the supply and return purge gas conduits having filters (22,23,25) to filter out particles from the purge gas being delivered to and recovered from the ante-chamber (10) respectively, said second valves (17,18) being disposed between the filters (22,23,25) and ante-chamber (10); the arrangement being such that the first and second valve means (14,16,17,18) are operable to periodically provide a flow of sterilant vapour to sterilise the purge gas supply and return conduits and filters.

2. An ante-chamber (10) as claimed in claim 1, **characterised in that** a third valve (21) is provided for controlling the supply of sterilant to the ante-chamber (10) to sterilise the purge gas supply and return conduits.

3. An ante-chamber (10) as claimed in claim 2, **characterised in that** the third valve (21) for controlling the supply of sterilant vapour to the ante-chamber (10) is located upstream of the filter (22) in the purge gas supply conduit.

4. An ante-chamber (10) as claimed in claim 2 or claim 3, **characterised in that** the purge gas return conduit has a catalyst (24) downstream of the filter (23) for converting the sterilant vapour into products which may be discharged to atmosphere.

5. An ante-chamber (10) as claimed in claim 4, **characterised in that** a further filter (25) is located in the purge gas return conduit downstream of the catalyst (24) to remove any particle in the purge gas received from the catalyst.

6. An ante-chamber (10) as claimed in any of claims 2-5, **characterised in that** the purge gas supply conduit has a fan (20) for delivering purge gas to the ante-chamber (10) via the filter (22) and third valve (21) to purge sterilant gas from the ante-chamber (10).

7. An ante-chamber (10) as claimed in any of the claims 1 to 6, **characterised in that** the purge gas return conduit has a fan (19) for extracting purge gas from the ante-chamber (10) disposed downstream of the second valve (17) and filter (23).

8. An ante-chamber (10) as claimed in any of the claims 1 to 6, **characterised in that** the purge gas supply and return conduits contain filters (22,23,25) and a catalyst (24) for converting sterilant vapour to harmless products disposed between the filters (22,23,25) and the second valves (17,18) are arranged to open both purge gas return and supply conduits for delivery of sterilant vapour from the ante-chamber (10) to sterilise the purge gas supply and return conduits.

9. An ante-chamber (10) as claimed in claim 1, **characterised in that** the purge gas supply and return conduits form a closed loop system.

10. An ante-chamber (10) as claimed in any of the preceding claims in combination with an isolator enclosure, a room or a cabinet.

## Patentansprüche

1. Vorkammer (10) zum Vorsterilisieren von Komponenten/ Materialien für die Versorgung eines geschlossenen Bearbeitungsraums, wobei die Vorkammer (10) Folgendes aufweist: einen verschließbaren Zugang (11) zum Aufnehmen von Komponenten/ Materialien, einen verschließbaren Auslass (12) zum Ausgeben von Materialien/Komponenten an den geschlossenen Bearbeitungsraum und erste Ventile (14, 16) zum Steuern der Zufuhr und Rückfuhr sterilisierenden Dampfes zur und aus der Vorkammer (10), um die Vorkammer (10) und deren Inhalt zu sterilisieren; **dadurch gekennzeichnet, dass** die Vorkammer (10) zweite Ventile (17,18) aufweist, um die Zufuhr und Rückfuhr von Spülgas zur und aus der Vorkammer (10) über die Zufuhr- und Rückfuhrleitung zum Ausspülen des Sterilisationsmittels aus der Vorkammer (10) nach Beendigung des Sterilisationsvorgangs zu steuern, wobei die Spülgaszufuhrleitung und die Spülgasrückfuhrleitung Filter (22, 23, 25) zum Ausfiltern von Partikeln aus dem Spülgas aufweisen, das der Vorkammer (10) zugeführt oder aus dieser zurückgeführt wird, und wobei die zweiten Ventile (17, 18) zwischen den Filtern (22, 23, 25) und der Vorkammer (10) angeordnet sind; und wobei die Anordnung zum Betrieb der ersten und der zweiten Ventileinrichtungen (14, 15, 17, 18) in einer Weise ausgebildet ist, dass regelmäßig eine sterilisierende Dampfströmung zum Sterilisieren der Spülgaszufuhrleitung und Spülgasrückfuhrleitung und der Filter zur Verfügung gestellt wird.

2. Vorkammer (10), wie in Anspruch 1 beansprucht,
**dadurch gekennzeichnet, dass** ein drittes Ventil (21) vorgesehen ist, um die Zufuhr des Sterilisationsmittels zur Vorkammer (10) für die Sterilisation von Spülgaszufuhrleitung und Spülgasrückfuhrleitung zu steuern.

3. Vorkammer (10), wie in Anspruch 2 beansprucht,
**dadurch gekennzeichnet, dass** das dritte Ventil (21) zum Steuern der Zufuhr sterilisierenden Gases zur Vorkammer (10) in der Spülgaszufuhrleitung stromaufwärts des Filters (22) angeordnet ist.

4. Vorkammer (10), wie in Anspruch 2 oder Anspruch 3 beansprucht,
**dadurch gekennzeichnet, dass** die Spülgasrückfuhrleitung stromabwärts des Filters (23) einen Katalysator (24) zum Umwandeln des sterilisierenden Dampfes in Produkte umfasst, die in die Atmosphäre abgegeben werden können.

5. Vorkammer (10), wie in Anspruch 4 beansprucht,
**dadurch gekennzeichnet, dass** in der Spülgasrückfuhrleitung stromabwärts des Katalysators (24) ein weiterer Filter (25) zum Entfernen jedweder Partikel aus dem vom Katalysator erhaltenen Spülgas angeordnet ist.

6. Vorkammer (10), wie in einem der Ansprüche 2 bis 5 beansprucht,
**dadurch gekennzeichnet, dass** die Spülgaszufuhrleitung ein Gebläse (20) für das Zuführen von Spülgas zur Vorkammer (10) über den Filter (22) und ein drittes Ventil (21) zum Ausspülen von sterilisierendem Gas aus der Vorkammer (10) aufweist.

7. Vorkammer (10), wie in einem der Ansprüche 1 bis 6 beansprucht,
**dadurch gekennzeichnet, dass** die Spülgasrückfuhrleitung ein stromabwärts des zweiten Ventils (17) und des Filters (23) angeordnetes Gebläse (19) zum Absaugen von Spülgas aus der Vorkammer (10) aufweist.

8. Vorkammer (10), wie in einem der Ansprüche 1 bis 6 beansprucht,
**dadurch gekennzeichnet, dass** die Spülgaszufuhrleitung und die Spülgasrückfuhrleitung Filter (22, 23, 25) und einen zwischen den Filtern (22, 23, 25) angeordneten Katalysator (24) zum Umwandeln sterilisierenden Dampfes in harmlose Produkte aufweisen, wobei die zweiten Ventile (17, 18) dazu ausgebildet sind, sowohl die Spülgasrückfuhrleitung als auch die Spülgaszufuhrleitung für ein Zuführen sterilisierenden Dampfes aus der Vorkammer (10) zum Sterilisieren der Spülgaszufuhrleitung und der Spülgasrückfuhrleitung zu öffnen.

9. Vorkammer (10), wie in Anspruch 1 beansprucht,
**dadurch gekennzeichnet, dass** die Spülgaszufuhrleitung und die Spülgasrückfuhrleitung ein System mit einem geschlossenen Kreislauf ausbilden.

10. Vorkammer (10), wie in einem der vorhergehenden Ansprüche beansprucht, kombiniert mit einem geschlossenen Isolator, einem Raum, oder einem Schrank.

## Revendications

1. Préchambre (10) pour la préstérilisation de composants/matériaux à amener à une enceinte de traitement, la préchambre (10) ayant une entrée (11) apte à être fermée pour la réception de composants/matériaux, une sortie apte à être fermée (12) pour l'amenée de matériaux/composants à l'enceinte de traitement et des premières vannes (14, 16) pour commander l'amenée et le retour d'une vapeur stérilisante vers et de la préchambre (10) pour stériliser la préchambre (10) et son contenu; **caractérisée en ce que** la préchambre (10) possède des deuxièmes vannes (17, 18) pour commander l'amenée et le retour d'un gaz de purge vers et de la préchambre (10) par des conduits d'amenée et de retour pour purger la préchambre (10) du stérilisant à la fin de l'opération de stérilisation, les conduits de gaz de purge d'amenée et de retour ayant des filtres (22, 23, 25) pour filtrer des particules du gaz de purge délivré à et récupéré de la préchambre (10), respectivement, lesdites deuxièmes vannes (17, 18) étant disposées entre les filtres (22, 23, 25) et la préchambre (10); l'agencement étant tel que les premier et deuxième moyens de vanne (14, 16, 17, 18) sont actionnables pour réaliser périodiquement un écoulement de vapeur stérilisante afin de stériliser les conduits d'amenée et de retour du gaz de purge et les filtres.

2. Préchambre (10) selon la revendication 1, **caractérisée en ce qu'**une troisième vanne (21) est réalisée pour commander l'amenée d'un stérilisant à la préchambre (10) pour stériliser les conduits d'amenée et de retour du gaz de purge.

3. Préchambre (10) selon la revendication 2, **caractérisée en ce que** la troisième vanne (21) pour commander l'amenée d'une vapeur stérilisante à la préchambre (10) est localisée en amont du filtre (22) dans le conduit d'amenée de gaz de purge.

4. Préchambre (10) selon la revendication 2 ou la revendication 3, **caractérisée en ce que** le conduit de retour de gaz de purge possède un catalyseur (24) en aval du filtre (23) pour convertir la vapeur stérilisante en produits qui peuvent être évacués dans l'atmosphère.

5. Préchambre (10) selon la revendication 4, **caractérisé en ce qu'**un autre filtre (25) est situé dans le conduit de retour du gaz de purge en aval du catalyseur (24) pour retirer toute particule dans le gaz de purge reçu du catalyseur.

6. Préchambre (10) selon l'une quelconque des revendications 2 à 5, **caractérisée en ce que** le conduit d'amenée de gaz de purge possède un ventilateur (20) pour délivrer du gaz de purge à la préchambre (10) par le filtre (22) et une troisième vanne (21) pour purger le gaz de stérilisation de la préchambre (10).

7. Préchambre (10) selon l'une quelconque des revendications 1 à 6, **caractérisée en ce que** le conduit de retour de gaz de purge possède un ventilateur (19) pour extraire le gaz de purge de la préchambre (10) disposé en aval de la deuxième vanne (17) et du filtre (23).

8. Préchambre (10) selon l'une quelconque des revendications 1 à 6, **caractérisée en ce que** les conduits d'amenée et de retour de gaz de purge contiennent des filtres (22, 23, 25) et un catalyseur (24) pour convertir la vapeur de stérilisation en produits inoffensifs disposés entre les filtres (22, 23, 25) et les deuxièmes vannes (17, 18) sont agencées pour ouvrir à la fois les conduits de retour et d'amenée du gaz de purge pour la délivrance de la vapeur de stérilisation de la préchambre (10) afin de stériliser les conduits d'amenée et de retour du gaz de purge.

9. Préchambre (10) selon la revendication 1, **caractérisée en ce que** les conduits d'amenée et de retour du gaz de purge forment un système en boucle fermée.

10. Préchambre (10) selon l'une quelconque des revendications précédentes, en combinaison avec une enceinte d'isolation, une pièce ou un boîtier.
